# EUROPÄISCHE PATENTSCHRIFT

(11) **EP 0 876 596 B1**
(45) Veröffentlichungstag und Bekanntmachung des Hinweises auf die Patenterteilung: **03.07.2002**
(21) Anmeldenummer: 97914097.7
(22) Anmeldetag: 23.01.1997
(51) Int. Cl.: G01N 21/49, A61B 5/00

(54) **VERFAHREN UND VORRICHTUNG ZUR BESTIMMUNG EINES ANALYTEN IN EINER STREUENDEN MATRIX**
PROCESS AND DEVICE FOR DETERMINING AN ANALYTE CONTAINED IN A SCATTERING MATRIX
PROCEDE ET DISPOSITIF DE DETERMINATION D'UN ANALYTE CONTENU DANS UNE MATRICE DE DIFFUSION

(30) Priorität: 26.01.1996 DE 19602656
(43) Veröffentlichungstag der Anmeldung: 11.11.1998
(73) Patentinhaber: Roche Diagnostics GmbH, 68298 Mannheim (DE)
(72) Erfinder: KNÜTTEL, Alexander, D-69488 Birkenau (DE); BOECKER, Dirk, D-69115 Heidelberg (DE); ESSENPREIS, Matthias, D-82131 Gauting (DE)
(74) Vertreter: Pfeifer, Hans-Peter, Dr.
(86) Internationale Anmeldenummer: DE9700168
(87) Internationale Veröffentlichungsnummer: WO9727469

(56) Entgegenhaltungen:
- EP-A- 0 707 826
- WO-A-92/19930
- DE-A- 4 337 570
- DE-A- 4 415 728
- US-A- 5 203 328
- US-A- 5 549 114

## Beschreibung

Die Erfindung betrifft ein Verfahren und eine Vorrichtung zur Analyse einer streuenden Matrix mit Hilfe von Licht auf einen darin enthaltenen Analyten.

Der wichtigste Anwendungsfall der Erfindung ist die analytische Untersuchung biologischer Proben, insbesondere des Gewebes eines lebenden Organismus. Biologische Proben sind meistens optisch heterogen, d.h. sie enthalten eine Vielzahl von Streuzentren, an denen eingestrahltes Licht gestreut wird. Dies gilt für menschliches oder tierisches Gewebe, insbesondere Hautgewebe oder subkutanes Fettgewebe, aber auch für flüssige biologische Proben, wie beispielsweise Blut, in dem die Blutkörperchen Streuzentren bilden oder auch für Milch, bei dem die Streuzentren von emulgierten Fetttröpfchen gebildet werden.

Daneben richtet sich die Erfindung generell auf streuende Matrices, in denen ein Analyt qualitativ oder quantitativ bestimmt werden soll. Eine streuende Matrix in diesem Sinne ist ein dreidimensionales Gebilde mit einer so hohen Dichte an optischen Streuzentren, daß eindringendes Licht in der Regel vielfach gestreut wird, bevor es die streuende Matrix wieder verläßt. Nichtbiologische streuende Matrices, die auf Basis der vorliegenden Erfindung untersucht werden können, sind beispielsweise Emulsionen und Dispersionen, wie sie für unterschiedliche Anwendungszwecke, beispielsweise für Lacke und Farben benötigt werden.

Nachfolgend wird ohne Beschränkung der Allgemeinheit auf die Analyse von Gewebe als Beispiel für biologische und andere streuende Matrices Bezug genommen.

Ziel der Analyseverfahren, auf die sich die Erfindung richtet, ist die Bestimmung eines Analyten in dem Sinn, daß eine Information über die Gegenwart einer bestimmten in dem Gewebe enthaltenen Komponente gewonnen wird. Die Information kann sich auf die Konzentration des Analyten beziehen (quantitative Analyse) oder auch nur auf die Frage, ob der Analyt (in einer über der Nachweisgrenze des Verfahrens liegenden Konzentration) in der Probe enthalten ist (qualitative Analyse).

Analysen von Geweben und anderen biologischen Proben werden bisher überwiegend invasiv durchgeführt, d.h. es wird eine Probe (meist eine Blutprobe) aus dem Gewebe entnommen, in der die Analytkonzentration mit Hilfe von Reagenzien bestimmt wird.

In neuerer Zeit werden in zunehmendem Maße nicht-invasive Analyseverfahren diskutiert, bei denen das analytische Ergebnis ohne Probenentnahme aus dem Gewebe schmerzfrei und reagenzienfrei gewonnen wird. Die meisten zu diesem Zweck diskutierten Verfahren basieren auf der Wechselwirkung von Licht mit der streuenden Matrix. Gemeinsam ist diesen Verfahren, daß Meßschritte durchgeführt werden, bei denen Licht durch eine die Matrix begrenzende Grenzfläche als Primärlicht in die Matrix eingestrahlt und aus der Matrix austretendes Licht als Sekundärlicht detektiert wird, um eine durch die Wechselwirkung des Lichts mit der Matrix veränderliche meßbare physikalische Eigenschaft des Lichtes zu messen, die mit der Konzentration des Analyten in der Matrix korreliert. Ein solcher Verfahrensschritt wird hier als "Detektionsschritt" und die Messung als "Detektionsmessung" bezeichnet, wobei ein Detektionsschritt eine oder mehrere Detektionsmessungen umfassen kann.

Die Wellenlängen des Lichts, die für solche Verfahren diskutiert werden, liegen im allgemeinen zwischen etwa 300 nm und mehreren tausend nm, also im Spektralbereich zwischen dem nahen UV- und Infrarotlicht. Die in dem Detektionsschritt bestimmte (detektierte) physikalische Eigenschaft des Lichtes, die man auch als "quantifizierbarer Parameter" (englisch: quantifiable parameter) bezeichnen kann, wird nachfolgend einfachheitshalber als "Meßgröße" bezeichnet.

Da bei den hier diskutierten Verfahren in aller Regel keine Absolutmessung der Analytkonzentration möglich ist, ist (ebenso wie bei den meisten auf chemischen Reaktionen basierenden Analyseverfahren) eine Kalibration erforderlich. Üblicherweise wird in mindestens einem Kalibrationsschritt, der meßtechnisch in gleicher Weise wie ein Detektionsschritt ausgeführt wird, mindestens eine Detektionsmessung an einer streuenden Matrix mit bekannter Analytkonzentration durchgeführt. Im Falle der Analyse lebenden Gewebes geschieht dies zweckmäßigerweise durch eine Vergleichsmessung mit irgendeinem vorbekannten Analyseverfahren.

In einem Auswerteschritt des Analyseverfahrens wird die Analytkonzentration aus der Änderung der Meßgröße bei mindestens einem Detektionsschritt im Vergleich zu mindestens einem Kalibrationsschritt bestimmt. Der Auswerteschritt umfaßt einen Auswertealgorithmus, in dem die Analytkonzentration in vorbestimmter Weise aus den Ergebnissen von mindestens einem Detektionsschritt und mindestens einem Kalibrationsschritt ermittelt wird.

Die meisten Verfahren dieser Art basieren auf den Prinzipien der Spektroskopie, d.h. auf der Untersuchung der spektralen Abhängigkeit der optischen Absorption. Zu diesem Zweck werden Detektionsmessungen bei mindestens zwei unterschiedlichen Lichtwellenlängen durchgeführt. Während dies in einer klaren Flüssigkeit ein seit langem etabliertes problemloses Verfahren ist, bestehen bei der spektroskopischen Analyse an Gewebe und anderen streuenden Matrices schwerwiegende Probleme.

Zum einen ist für die meisten Analyten das Nutzsignal (die Änderung des Absorptions-Spektrums in Abhängigkeit von einer Änderung der Analytkonzentration) sehr klein und diesem kleinen Nutzsignal steht ein großer Hintergrund von Störsignalen gegenüber, die insbesondere von der optischen Absorption von Wasser und anderen stark absorbierenden Komponenten (unter anderem dem roten Blutfarbstoff Hämoglobin) resultieren.

Zum zweiten besteht in einer streuenden Matrix aufgrund der Vielfachstreuung des Lichts das Problem, daß die von dem Licht in der Probe zurückgelegte optische Weglänge unbekannt ist. Die Kenntnis dieser Weglänge ist jedoch Voraussetzung, um bei einer spektroskopischen Analyse gemäß dem Gesetz von Lambert-Beer die Konzentration eines Analyten bestimmen zu können.

Zur Lösung dieser Probleme wurden zahlreiche unterschiedliche Versuche unternommen. Insbesondere werden Vergleichstechniken angewendet, bei denen der Versuch gemacht wird, die Einflüsse von stark absorbierenden störenden Substanzen und auch den Einfluß der Vielfachstreuung und der daraus resultierenden fehlenden Kenntnis der optischen Weglänge durch mehrere Detektionsmessungen und Verhältnis- oder Differenzbildung zu eliminieren. Speziell mit dem Problem der unbekannten optischen Weglänge befaßt sich die zeitaufgelöste Spektroskopie.

Trotz dieser Bemühungen hat die spektroskopische Analyse von Gewebe nur für einen Analyten, nämlich den roten Blutfarbstoffes Hämoglobin (Hb bzw. dessen oxidierte Form HbO₂) praktische Bedeutung erlangt. Diese Substanzen absorbieren so stark und sind in so hoher Konzentration vorhanden, daß eine spektroskopische Bestimmung mit den bekannten Verfahren möglich ist. Gerade diese starke optische Absorption ist jedoch ein wesentlicher Grund, warum die spektroskopische Analyse anderer Analyten bisher erfolglos war.

Ein besonders wichtiger Analyt ist die Glucose, weil eine langfristig erfolgreiche Therapie von Diabetikern eine möglichst kontinuierliche Überwachung des Glucosespiegels im Körper voraussetzt. Um schwere Spätschäden, wie beispielsweise Erblindung oder Amputation von Gliedmaßen zu vermeiden, muß die Glucosekonzentration mindestens fünfmal täglich bestimmt werden. Dies ist mit invasiven Verfahren kaum möglich.

Die optische Absorption des Gewebes hängt jedoch nur in einem sehr geringen Ausmaß von der Glucosekonzentration ab. Deswegen haben spektroskopische Prinzipien nicht zum Erfolg geführt. Statt dessen werden verschiedene alternative Verfahren zur nicht-invasiven Bestimmung der Glucosekonzentration diskutiert.

Beispielsweise wird in der europäischen Patentschrift 0074428 davon ausgegangen, daß die Glucosemoleküle einen durch eine Glucoselösung transmittierten Lichtstrahl in charakteristischer Weise streuen und sich aus der Raumwinkelverteilung der aus einer Untersuchungsküvette oder aus einem untersuchten Körperteil austretenden transmittierten Lichtintensität die Glucosekonzentration ermitteln läßt. In der WO 94/10901 wird die räumliche Verteilung der Sekundärlichtintensität an der. Grenzfläche als Maß für die Glucosekonzentration bestimmt und es wird erläutert, daß diese räumliche Verteilung in charakteristischer Weise von der Glucosekonzentration in einer Gewebeprobe abhängt, weil die Glucosekonzentration aufgrund der Vielfachstreuung in dem Gewebe die räumliche Verteilung des aus der Grenzfläche austretenden Sekundärlichts in überraschend starkem Ausmaß beeinflußt. In der DE 4243142 A1 wird ein Verfahren zur Bestimmung der Glucosekonzentration in der Vorderkammer des Auges beschrieben, wobei als Meßgrößen die optische Absorption und die Rotation von polarisiert eingestrahltem Licht beschrieben werden. In der WO 95/30368 und der DE 4415728 A1 werden verschiedene Möglichkeiten beschrieben, die Glucosekonzentration auf Basis von LCI (Low Coherence Interferometry)-Messungen zu bestimmen, wobei als Meßgrößen unter anderem der Streukoeffizient und der Brechungsindex, die beide von der Glucosekonzentration abhängen, diskutiert werden.

Die in diesen Publikationen beschriebenen Verfahren bilden zwar bedeutsame Ansätze zur Lösung der mit der nicht-invasiven Analytik in Gewebeproben verbundenen Probleme. Sie sind jedoch auf einen bestimmten Analyten, nämlich die Glucose, beschränkt. Außerdem ist die Selektivität prinzipbedingt relativ gering.

Auch die US-Patentschrift 5,203,328 befaßt sich mit einem diagnostischen Verfahren im Zusammenhang mit dem Diabetes Mellitus. Hier geht es allerdings nicht um die Analyse der Glucosekonzentration im Körper des Patienten zur Steuerung der Insulin-Therapie, sondern um die Früherkennung der Erkrankung. Die Literaturstelle beschäftigt sich damit, daß bei Diabetikern häufig eine Trübung der Augenlinse festzustellen ist und daß die Messung dieser Trübung als Indiz für das Vorliegen der Erkrankung geeignet sein könnte. Voraussetzung sei allerdings, daß die Diabetes-relevante Trübung von anderen Trübungsursachen, insbesondere im Hinblick auf das Alter der meisten Patienten, unterschieden werden kann. Um diese Differenzierung zu ermöglichen, wird eine bestimmte Kombination der Messung von Fluoreszenzlicht und Rayleigh-Streuung vorgeschlagen, bei der eine confokale Meßanordnung verwendet und die Untersuchungsstelle mittels eines zusätzlichen Okulares beobachtet wird.

Der Erfindung liegt die Aufgabe zugrunde, ein Verfahren und eine Vorrichtung zur Analyse von Gewebe oder anderen optisch stark streuenden Matrices zur Verfügung zu stellen, welches es erlaubt, einen darin enthaltenen Analyten selbst dann selektiv zu analysieren, wenn die totale optische Absorption der streuenden Matrix nur zu einem sehr geringen Anteil von der Konzentration des Analyten beeinflußt wird.

Die Aufgabe wird durch ein Verfahren nach Anspruch 1 und eine Vorrichtung nach Anspruch 13 gelöst.

Eine confokale Anordnung, bei der ein Objekt mit auf einen Brennpunkt (Fokus) fokussiertem Licht beleuchtet und die Beobachtung auf den gleichen Fokus konzentriert wird, ist für verschiedene Anwendungszwecke, darunter auch spektroskopische Messungen, beispielsweise aus den nachfolgenden Zitaten bekannt:
1) C.J.R. SHEPPARD et al.: "Imaging performance of confocal fluorescence microscopes with finite-sized source", Journal of Modern Optics, 41 (1994), pp. 1521-1530.
2) US-Patent 5,192,980
3) US-Patent 5,345,306
4) EP 0689045

Da das Licht bei der confokalen Anordnung auf den Fokusbereich in einer bestimmten Fokustiefe konzentriert wird und auch die Detektion sich auf den gleichen Fokusbereich konzentriert, bewirkt die confokale Anordnung eine gewisse Tiefenselektion. Der Detektor erfaßt vor allem Photonen, die in einem Abstand von der Grenzfläche reflektiert werden, der der Fokustiefe der confokalen Anordnung entspricht. Um die vorteilhaften Ergebnisse der vorliegenden Erfindung zu erreichen, ist es jedoch notwendig, ein zusätzliches Tiefenselektionsmittel einzusetzen, durch das selektiv aus einer mit der Fokustiefe übereinstimmenden definierten Meßtiefe reflektiertes Licht als Sekundärlicht detektiert wird. Hierzu ist ein timegating-Verfahren geeignet, wie es (für einen anderen Anwendungszweck, nämlich zur Bestimmung des Brechungsindex der Probe auf Basis einer Messung der Laufzeit des Lichts) beispielsweise aus der DE 4337570 D1 bekannt ist.

Besonders bevorzugt ist jedoch eine zusätzliche Tiefenselektion mit Hilfe eines Niederkohärenz-reflektometrischen Meßverfahrens. Auch solche Verfahren sind für andere Anwendungen bekannt, beispielsweise zur Bestimmung des Brechungsindex oder des Streukoeffizienten als Maß für die Glucosekonzentration (aus der weiter oben erwähnten DE 4415728 A1) und zum Zwecke einer tomographischen Bilddarstellung (sogenannte "optical coherence tomography", vgl. WO 92/19930).

Die confokale Anordnung führt dazu, daß Photonen, die auf dem Weg von dem Fokus zu der Grenzfläche in der Matrix gestreut und dadurch abgelenkt werden, nicht durch die in dem Lichtweg vor dem Detektor angeordnete Lichteintrittsöffnung zu dem Detektor gelangen können. Zu dem Signal des Detektors tragen deshalb überwiegend Photonen bei, die an einer Struktur in dem Bereich des Fokus in der streuenden Matrix reflektiert werden und die von dort ungestreut die Matrix verlassen.

In der Praxis ist es nicht möglich das Primärlicht auf einen geometrischen Punkt in der Matrix zu fokussieren und die Abbildung auf den gleichen geometrischen Punkt zu konzentrieren. Optische Abbildungsfehler und die endliche Größe der Lichtquelle sowie der detektorseitigen Lichteintrittsöffnung führen vielmehr unvermeidlich dazu, daß das mit der confokalen Anordnung erfaßte Teilvolumen der Matrix eine endliche Größe hat. Dieses Teilvolumen wird als "Fokusbereich" bezeichnet.

Bei Verwendung des erfindungsgemäßen Verfahrens und der entsprechenden Vorrichtung führen Änderungen der Konzentration eines Analyten, der einen sehr geringen Anteil an der optischen Gesamtabsorption hat, zu verhältnismäßig starken Änderungen des Meßsignals. Dadurch ist eine selektive Analyse auch solcher problematischer Analyten möglich. Dies gilt insbesondere in einem Wellenlängenbereich, in dem der Analyt eine Absorptionsbande hat.

Die Erfindung wird nachfolgend anhand von in den Figuren dargestellten Ausführungsbeispielen näher erläutert. Es zeigen:
- Fig. 1: Eine Prinzipdarstellung einer erfindungsgemäßen Analysevorrichtung,
- Fig. 2a und 2b: Schaubilder der Abhängigkeit des Phasen-Brechungsindex von der Lichtwellenlänge für zwei unterschiedliche Konzentrationen einer Glucoselösung in Wasser,
- Fig. 3: ein Balkendiagramm des Gruppen-Brechungsindex von Glucose und von zwei Störsubstanzen jeweils gelöst in Wasser für vier unterschiedliche Wellenlängen des Lichts,
- Fig. 4: den differentiellen Gruppen-Brechungsindex für die Substanzen von Figur 3 und je zwei Wellenlängen-Paare,
- Fig. 5: einen Teil einer alternativen Ausführungsform einer Analysevorrichtung in einer Prinzip-Schnittdarstellung,
- Fig. 6: eine Prinzip-Schnittdarstellung von einer weiteren Ausführungsform,
- Fig. 7: eine Prinzip-Schnittdarstellung einer weiteren Ausführungsform,
- Fig. 8 und 9: zwei Prinzip-Schnittdarstellungen unterschiedlicher Ausführungsformen zur Realisierung einer spektral aufgelösten Messung bei mehreren Lichtwellenlängen,
- Fig. 10: eine Prinzip-Schnittdarstellung einer auf Basis von Fig. 7 weitergebildeten Ausführungsform.

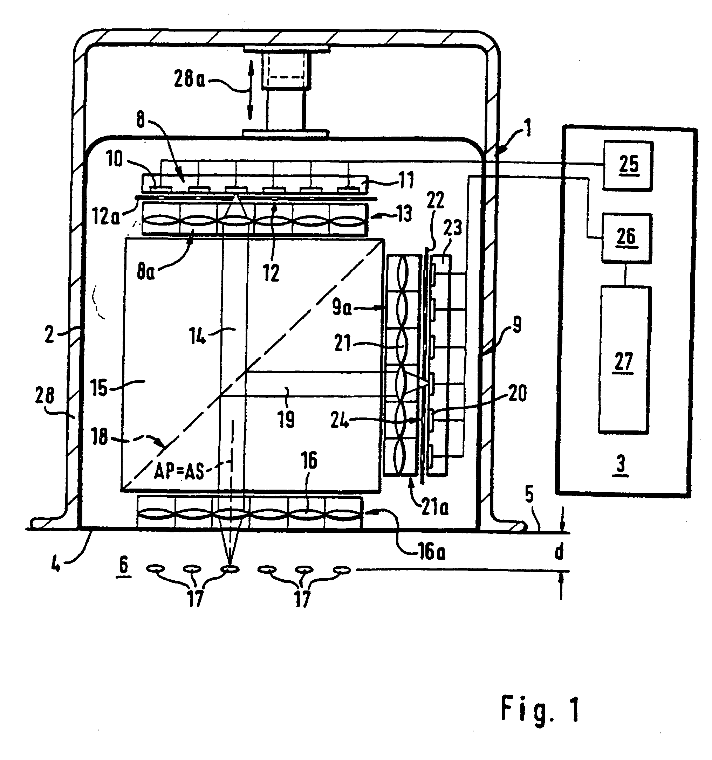

Die in Figur 1 stark schematisiert, teilweise im Schnitt und teilweise als Blockdiagramm, dargestellte Analysevorrichtung 1 besteht im wesentlichen aus einem Meßkopf 2 und einer Elektronikeinheit 3. Der Meßkopf 2 liegt mit einer Probenkontaktfläche 4 an der Grenzfläche 5 einer streuenden Matrix 6 (z.B. an der Oberfläche menschlicher Haut) an. In dem Meßkopf 2 befinden sich Lichteinstrahlungsmittel 8 zum Einstrahlen von Primärlicht 14 in die Matrix 6 und Detektionsmittel 9 zum Detektieren von aus der Matrix 6 austretendem Sekundärlicht 19.

Die Lichteinstrahlungsmittel 8 umfassen mehrere Lichtsender 10, denen jeweils ein (im dargestellten Fall durch die Linsen 13 und 16 gebildetes) optisches System 8a zugeordnet ist, durch das das Primärlicht 14 auf einen in dem Gewebe in einer vorbestimmten Tiefe d der Matrix liegenden Fokusbereich 17 fokussiert wird. In der dargestellten Ausführungsform sind mehrere Halbleiterlichtsender (Leuchtdioden) in einem Halbleitersubstrat (Chip) 11 monolitisch integriert. Um eine gute Fokussierung zu ermöglichen, sollten die Lichtsender 10 möglichst punktförmig sein. Zur Begrenzung der Lichtaustrittsöffnungen 12 befinden sich Lochblenden 12a vor den Lichtsendern 10.

Das von den Lichtaustrittsöffnungen 12 ausgehende divergierende Licht wird durch eine Anordnung von Kollimationslinsen 13 kollimiert und tritt als Parallelstrahlenbündel in einen Strahlteilerwürfel 15 (beam splitter cube BSC) ein. Das aus der gegenüberliegenden Fläche des BSC 15 austretende Licht wird durch eine Fokussierungslinse 16 auf den Fokusbereich 17 fokussiert.

Die Lichteinstrahlungsmittel, bestehend aus den Lichtsendern 10 und den Komponenten 12,13 und 16 sind mehrfach (n-fach) vorhanden und als (bevorzugt regelmäßiges) Array parallel zu der Grenzfläche 5 der Matrix 6 derartig angeordnet, daß Primärlichtstrahlen 14 auf eine Mehrzahl von Fokusbereichen 17, die bevorzugt in gleicher Meßtiefe d in dem Gewebe 6 liegen, fokussiert werden. In Figur 1 ist der Strahlengang der Übersichtlichkeit halber nur für einen der Fokusbereiche 17 dargestellt.

Das von jeweils einem der Fokusbereiche 17 ausgehende Sekundärlicht wird durch die Linsen 16 kollimiert und fällt koaxial in den Primärstrahl zurück. In dem BSC 15 erfolgt eine Strahlteilung, wobei das Sekundärlicht senkrecht zu der Richtung des Primärlichtstrahls 14 in Richtung auf einen Detektor 20 reflektiert wird. Vor dem Detektor 20 befindet sich eine weitere Linse 21 und eine Lochblende 22, die die detektorseitige Lichteintrittsöffnung 24 bildet. Die Linsen 16 und 21 bilden insgesamt ein optisches Abbildungssystem 9a, durch die der Fokusbereich 17 auf die Lichteintrittsöffnung 24 (genauer gesagt auf die Ebene der detektorseitigen Lichteintrittsöffnung 24) abgebildet wird.

Die Detektoren 20 sind in gleicher Anzahl wie die Lichtsender 10 n-fach vorhanden und als Array entsprechend den Lichtsendern angeordnet, wobei jedem Detektor 20 ein optisches System 16,21 zugeordnet ist. Bevorzugt werden Halbleiterdetektoren (beispielsweise Avalanche-Photodioden) verwendet, die auf einem gemeinsamen Halbleitersubstrat 23 monolitisch integriert sind. Die Kollimatorlinsen 13, die Fokussierungslinsen 16 und die vor den Detektoren 20 angeordneten Abbildungslinsen 21 sind vorzugsweise jeweils als "Mikrolinsen-Array" (16a,21a) ausgebildet. Derartige Mikrolinsen-Arrays sind kommerziell erhältlich.

Die optischen Systeme 8a und 9a, welche Bestandteil der Lichteinstrahlungsmittel 8 und der Detektionsmittel 9 sind, können in unterschiedlicher Weise so aufgebaut sein, daß die erläuterten Beleuchtungs- und Abbildungsbedingungen erreicht werden. Insbesondere können statt der dargestellten einfachen Linsen auch jeweils mehrlinsige Systeme (Objektive), grundsätzlich auch Spiegel, als fokussierende Elemente eingesetzt werden.

Durch die Lichteinstrahlungsmittel 8 wird unter Verwendung eines optisch fokussierenden Elementes (hier der Fokussierungslinse 16) ein Lichtfokus in dem Fokusbereich 17 in der streuenden Matrix generiert. Die konfokale Anordnung der Detektionsmittel 9 führt dazu, daß (wie oben erläutert) überwiegend in dem Fokusbereich 17 reflektierte Photonen von den Detektoren 20 erfaßt werden und diffus gestreutes Licht nur zu einem geringen Anteil zu dem Detektor 20 gelangt.

Die Elektronikeinheit 3 enthält eine Stromversorgungsschaltung 25 für die Lichtsender 10, eine Verstärkerschaltung 26 zur Verstärkung der Ausgangssignale der Detektoren 20 und eine üblicherweise mit Hilfe eines Mikroprozessors realisierte Auswerteeinheit 27, die die gewünschte analytische Information aus den Meßsignalen der Detektoren 20 ermittelt.

Um mit Hilfe eines "time-gating"-Verfahrens ein zusätzliches Mittel zur Tiefenselektion (d.h. zum selektiven Erfassen von Photonen, die in einer mit der Fokustiefe d übereinstimmenden Meßtiefe reflektiert wurden) zur Verfügung zu stellen, erzeugt die Stromversorgungsschaltung 25 extrem kurze Signalimpulse, die von den Lichtsendern 10 in sehr kurze Lichtimpulse umgesetzt werden. Die Verstärkerschaltung 26 und die Auswerteeinheit 27 sind dafür eingerichtet, das von den Detektoren 20 erfaßte Sekundärlicht innerhalb eines definierten Zeitfensters, welches der gewünschten Meßtiefe d entspricht, zu erfassen. Die hierzu erforderlichen Mittel sind in Anbetracht der extrem kurzen Lichtlaufzeiten und der erforderlichen Präzision zwar aufwendig, jedoch praktisch verfügbar. Eine erforderliche Steuerleitung zur Übermittlung eines TriggerSignals ist in Figur 1 mit 25a bezeichnet. Nähere Einzelheiten über unterschiedliche für derartige Messungen geeignete Technologien können der einschlägigen Literatur entnommen werden. Verwiesen sei insbesondere auf folgende Publikationen:
5) "Femtosecond optical ranging in biological systems", von J. G. Fujimoto et al., OPTICS LETTERS, 1986, 150-152
6) "Time-resolved Fourier spectrum and imaging in highly scattering media", von L. Wang et al., APPLIED OPTICS, 1993, 5043 ff".
7) "A continuously variable frequency cross-correlation phase fluorometer with picosecond resolution", von E. Gratton et al., BIOPHYSICAL JOURNAL, 1983, 315-324

Bevorzugt werden bei der Erfindung mehrere Messungen mit unterschiedlichen Fokustiefen d durchgeführt. Zu diesem Zweck ist der Meßkopf 2 in Richtung senkrecht zu der Grenzfläche 5 bewegbar. Zweckmäßigerweise kann diese Vertikalpositionierung mittels eines auf der Grenzfläche 5 der streuenden Matrix 6 abgestützten rahmenförmigen Halteteils 28 und eines Positionierantriebs bewirkt werden, der in Figur 1 symbolisch als Doppelpfeil 28a dargestellt ist.

Die Kombination der erfindungsgemäßen meßtechnischen Maßnahmen führt dazu, daß zu dem Meßsignal der Detektoren 20 im wesentlichen nur Photonen beitragen, die in einer definierten Meßtiefe d reflektiert wurden und von dort im wesentlichen ungestreut zu dem jeweiligen Detektor 20 gelangen. Ungestreute Photonen werden auch als "ballistische" Photonen bezeichnet. Die erfindungsgemäße Anordnung erfaßt jedoch auch Photonen, die mit einem kleinen mittleren Streuwinkel gestreut werden und deren Bahn (propagation path) in der Nähe des geometrischen Lichtweges verläuft, die also nur minimal von der kürzesten (ballistischen) Bahn abweichen. Derartige Photonen werden als "quasiballistisch" bezeichnet. Die erfindungsgemäße Anordnung kann man deshalb als "tiefenselektiv-quasiballistisches Meßregime" bezeichnen.

Für die mit diesem tiefenselektiv-quasiballistischen Meßregime verbundenen Vorteile bei der Bestimmung eines Analyten in einer streuenden, insbesondere biologischen, Matrix lassen sich nach dem gegenwärtigen Kenntnisstand der Erfinder folgende Gründe angeben.

Die ballistischen oder quasiballistischen Photonen legen in der Probe die kürzestmögliche Wegstrecke zurück und werden deswegen relativ wenig absorbiert. Deswegen stören in der Probe enthaltene optisch stark absorbierende Störsubstanzen (im Falle von Gewebeproben vor allem Hämoglobin und Wasser) die Messung verhältnismäßig wenig. Mit diesem Effekt hängt auch zusammen, daß das Meßlicht trotz der starken Absorption dieser Störsubstanzen verhältnismäßig tief in die Probe eindringen kann und dadurch beispielsweise in der Haut Schichten erreicht werden, in denen sich die Konzentration der Glucose signifikant ändert. Weiterhin führt die geradlinige Bahn der bei dem erfindungsgemäßen Verfahren erfaßten Photonen dazu, daß die Länge des optischen Lichtweges in der Probe definiert ist. Sie entspricht der doppelten Meßtiefe d.

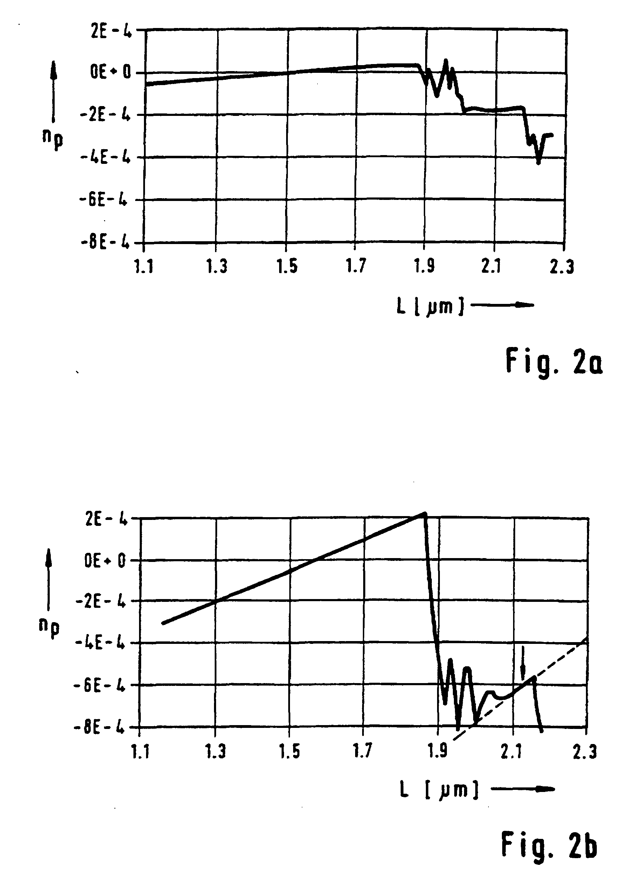

Von besonderer Bedeutung für die mit der Erfindung erzielten Vorteile ist ein Effekt, der nachfolgend anhand der Figuren 2 bis 4 näher erläutert wird. Unter den bei der Erfindung vorherrschenden Meßbedingungen wird die Schwächung der eingestrahlten Lichtintensität überwiegend von dem Streukoeffizienten µₛ und nur zu einem geringeren Anteil von dem Absorptionskoeffizienten µₐ bestimmt. Dabei ist zu berücksichtigen, daß im Gegensatz zu einem diffusen Meßregime, bei dem mit der erfindungsgemäßen Anordnung erfaßten tiefenselektiv-quasiballistischen Meßregime der Lichttransport nicht durch den korrigierten Streukoeffizienten µₛ', sondern durch den unkorrigierten Streukoeffizienten µₛ beschrieben wird, der stets größer ist als µₛ'. Im Rahmen der Erfindung wurde gefunden, daß der unkorrigierte Streukoeffizient und somit das Meßsignal unter den erfindungsgemäßen Meßbedingungen verhältnismäßig stark von der Konzentration des Analyten abhängt. Deshalb ist die Analyt-spezifische Signaländerung bei der Erfindung verhältnismäßig groß.

Die Figuren 2a und 2b zeigen den Verlauf des Phasen-Brechungsindex nₚ einer Glucoselösung in Wasser bei einer Glucosekonzentration von 6,25 mmol (Millimol pro Liter) bzw. 100 mmol in Abhängigkeit von der Wellenlänge L in µm. Diese Meßergebnisse wurden mit einem Interferometer gewonnen, mit dem die Abhängigkeit der Phase von der aus der Bewegung des Interferometerspiegels resultierenden Dopplerfrequenz bestimmt werden kann. Die in Figur 2a und 2b dargestellte Abhängigkeit des Phasen-Brechungsindex von der Wellenlänge läßt sich hieraus unmittelbar berechnen. Von Interesse ist insbesondere die (in Fig. 2b gestrichelt eingezeichnete) Steigung der dargestellten Kurve im Bereich des Absorptionsmaximums bei 2,15 µm. Diese Steigung entspricht dem Gruppen-Brechungsindex n_{G}, der das Streuverhalten in der streuenden Matrix bestimmt.

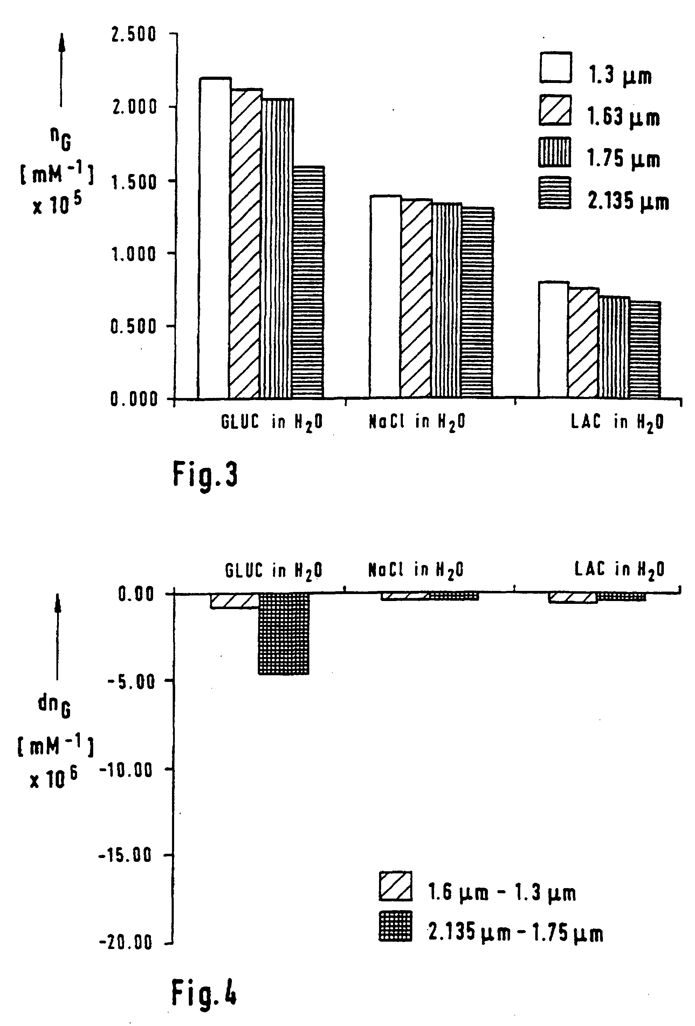

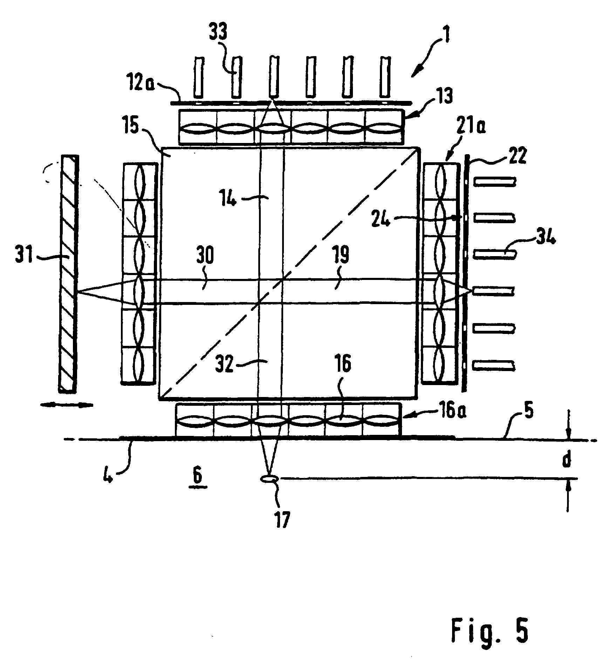

In Figur 3 ist in Form von Balkendiagrammen für drei verschiedene Substanzen, nämlich den Analyt Glucose (GLUC) und die zwei wichtigen Störsubstanzen NaCl und Lactat (LAC) der Gruppen-Brechungsindex n_{G} für jeweils vier verschiedene Lichtwellenlängen aufgetragen. Figur 4 zeigt den differentiellen Brechungsindex, bezogen auf die Wellenlänge. Aufgetragen sind die Gruppen-Brechungsindex-Differenzen dn_{G} für die drei Substanzen und für die Wellenlängenpaare 1,6 µm minus 1,3 µm sowie 2,135 µm minus 1,75 µm.

Die in Figur 3 dargestellten Absolutwerte des Gruppen-Brechungsindex n_{G} sind für die Glucose am höchsten, jedoch auch für die Störsubstanzen NaCl und Lactat erheblich. Eine wesentlich deutlichere Differenzierung zeigt der in Figur 4 dargestellte differentielle Brechungsindex. Insbesondere für das dargestellte Wellenlängenpaar 2,135 µm minus 1,75 µm ergibt sich beispielsweise ein Wert für dn_{G} von 5x10⁻⁶/mmol. In diesem Bereich befindet sich ein Absorptionsmaximum der Glucose in Wasser. Aus publizierten Literaturdaten läßt sich entnehmen, daß der differentielle Absorptionskoeffizient (d.h. die Änderung der Absorption in Abhängigkeit von einer Änderung der Glucosekonzentration) dµₐ = 2x10⁻⁴/mmol (für eine Wellenlänge L = 2,15 µm) beträgt.

In einer streuenden Matrix hängt die Lichtstreuung, d.h. der Streukoeffizient µₛ von dem Brechungsindex, der Größe und der Konzentration der streuenden Teilchen sowie von dem Brechungsindex des Mediums ab, in dem die streuenden Teilchen verteilt sind. Wenn diese Größen bekannt sind, läßt sich µₛ mit Hilfe der Mie-Theorie berechnen, wobei für derartige Berechnungen Computerprogramme verfügbar sind. Im Falle von Hautgewebe als streuende Matrix können folgende Näherungswerte angenommen werden:
- Brechungsindex der Streukörper (Zellen): 1,41
- Teilchengröße: 10 µm
- Konzentration: 5%
- Brechungsindex der interstitiellen Flüssigkeit: 1,38.

Hieraus resultiert gemäß der Mie-Rechnung ein Streukoeffizient von 6,8 mm⁻¹, d.h. ein mit Meßergebnissen übereinstimmender Wert für den (unkorrigierten) Streukoeffizienten µₛ in Gewebe. Dies bestätigt, daß die angenommenen Zahlenwerte sinnvoll sind. Aus dem oben genannten Wert des Brechungsindex-Unterschieds (differentiellen Brechungsindex) dn_{G} = 5x10⁻⁶/mmol resultiert gemäß der Mie-Rechnung ein entsprechender differentieller Streukoeffizient dµₛ = 2,015x10⁻³/ mmol. Dieser Wert ist 10mal so hoch wie die erwähnte differentielle Änderung des Absorptionskoeffizienten (dµₐ = 2x10⁻⁴/mmol).

Damit ist gezeigt, daß die erfindungsgemäße Meßanordnung eine Meßgröße erfaßt, die empfindlicher auf Änderungen der Analytkonzentration reagiert, als die konventionell gemessene optische Absorption in einem diffusen Meßregime gemäß dem Stand der Technik.

Die Meßbedingungen und das Verfahren der Auswertung können unter Berücksichtigung der erfindungsgemäßen Erkenntnisse optimiert werden, wobei folgende Überlegungen berücksichtigt werden sollten.

Bevorzugt werden im Rahmen der Erfindung mindestens zwei Detektionsmessungen mit unterschiedlichen Lichtwellenlängen durchgeführt. Dabei wird in dem Auswerteschritt vorteilhafterweise ein Quotient der Meßwerte bei den zwei unterschiedlichen Wellenlängen bestimmt und die Information über die Gegenwart des Analyten auf Basis des Quotienten ermittelt. Dadurch kann eine störende Hintergrundabsorption weitgehend eliminiert werden, wenn sich die gesamte optische Absorption der Probe sehr viel weniger als der Streukoeffizient ändert. Dies kann damit erklärt werden, daß die Gesamtschwächung der Lichtintensität in Abhängigkeit von der Meßtiefe d im Falle ballistischer Photonen proportional zu e^{-(µs+µa)d} ist. Hieraus folgt, daß µₐ eliminiert werden kann, wenn ein Quotient aus den Intensitätsmeßwerten von zwei Messungen mit konstanten µₐ gebildet wird.

Bei der Wahl der Meßtiefe d sind im Falle von Hautgewebe medizinische Belange zu berücksichtigen. Um eine Gewebeschicht zu erfassen, deren Glucosekonzentration medizinisch aussagekräftig ist, sollte die Meßtiefe mindestens etwa 0,3 mm betragen. Größere Meßtiefen führen wie beschrieben zu einer exponentiellen Abnahme der Intensität des Meßsignals. Gegenwärtig wird an Hautgewebe eine Meßtiefe von etwa 1,5 mm als maximale Obergrenze angesehen.

Vorzugsweise werden mindestens zwei Messungen durchgeführt, bei denen die mit der Fokustiefe d übereinstimmende Meßtiefe unterschiedlich ist, wobei besonders bevorzugt bei den beiden unterschiedlichen Meßtiefen d₁ und d₂ jeweils Messungen mit den gleichen Lichtwellenlängen L₁ und L₂ durchgeführt werden. Dadurch lassen sich in vorteilhafter Weise Meßfehler vermeiden, die auf Schwankungen der Intensität des eingestrahlten Primärlichtes I₀ zurückzuführen sind. Solche Schwankungen sind nicht nur infolge von Intensitätsschwankungen der Lichtsender 10 zu befürchten. Bei Messungen an der Haut stellt vielmehr der Übergang des Lichtes aus dem Meßkopf 2 durch die Grenzfläche 5 in die Probe 6 ("Ankopplung") ein grundlegendes Problem dar. Bereits kleine Änderungen der Position des Meßkopfes können Intensitätsänderungen des in die Probe 6 eingestrahlten Primärlichtes verursachen, die größer sind als das Meßsignal. Diese Meßfehler können bei der erfindungsgemäßen Meßanordnung eliminiert werden, wenn mit zwei unterschiedlichen Meßtiefen Messungen durchgeführt und ein Quotient der dabei gemessenen Intensitäts-Signale (jeweils bei der gleichen Wellenlänge) gebildet wird.

Dabei ist es besonders vorteilhaft, wenn eine der beiden Messungen mit einer möglichst geringen Meßtiefe und die zweite Messung mit einer möglichst großen Meßtiefe durchgeführt wird. Die kleine Meßtiefe sollte dabei unterhalb der Epidermisschicht liegen. Als Richtwert kann ein Bereich zwischen 0,3 mm und 0,5 mm angegeben werden. Die maximale Größe der zweiten (größeren) Meßtiefe ist im wesentlichen von der Intensität des Meßsignals bestimmt. Die Differenz beider Meßtiefen sollte mindestens 0,3 mm betragen.

Von Bedeutung ist auch die Größe der Lichteintrittsöffnung vor dem Detektor. In der Fachliteratur wurde der Begriff "wahre konfokale Abbildung (true confocal imaging)" geprägt, um Idealbedingungen einer konfokalen Abbildungsanordnung zu beschreiben. Näheres hierzu ist dem weiter oben zitierten Artikel von Sheppard et al. zu entnehmen. Im Rahmen der Erfindung wird bevorzugt mit einer deutlich größeren Lichteintrittsöffnung gearbeitet. Vorzugsweise sollte sie höchstens fünfmal so groß sein, wie für eine wahre konfokale Abbildung (gemäß den in dem Artikel von Sheppard et al. angegebenen Formeln) erforderlich. Generell sollte der Durchmesser der Lichteintrittsöffnung vor dem Detektor unter 0,1 mm, bevorzugt unter 0,05 mm liegen.

In der Praxis ist es außerordentlich vorteilhaft, nicht nur mit einer einzigen konfokalen Anordnung und einem einzigen Fokusbereich zu arbeiten, sondern eine Vielzahl von konfokalen Anordnungen in Form eines "Array" zu verwenden, durch das das Primärlicht auf eine Vielzahl von Fokusbereichen in der streuenden Matrix fokussiert und diese Fokusbereiche jeweils auf einen Detektor durch je ein Blendenloch abgebildet werden. Dadurch wird eine Erhöhung der Gesamt-Lichtintensität bei aus medizinischen Gründen vorgegebener maximaler Leistungsdichte erreicht. Außerdem werden Meßfehler durch Mikroheterogenitäten in der Haut durch Mittelwertbildung weitgehend eliminiert.

Die gewünschte analytische Information wird wie erwähnt mit Hilfe eines Auswertealgorithmus bestimmt, der die Meßwerte der gemessenen Meßgröße auf Basis einer Kalibration mit Konzentrationswerten verknüpft. Im vorliegenden Fall kann diese Verknüpfung in einer einfachen eindimensionalen Auswertekurve bestehen, die dem Quotienten aus den Intensitätsmeßwerten zweier Wellenlängen bzw. zweier Meßtiefen jeweils einen Konzentrationswert zuordnet.

In jüngerer Zeit werden in zunehmendem Umfang mathematisch aufwendigere Verfahren eingesetzt, um in der Analysetechnik die Korrelation zwischen den Meßgrößen (Eingangsvariablen) und der gesuchten Konzentration (Ausgangsvariable) zu verbessern und dadurch eine höhere Analysegenauigkeit zu erreichen. Hierzu gehören insbesondere auch multilineare und nichtlineare Algorithmen, die mehrere Faktoren miteinander verknüpfen, die für die Auswertung der analytischen Messung erforderlich sind. Im vorliegenden Fall kann es beispielsweise zweckmäßig sein, in jedem Meßschritt eine Vielzahl von Messungen bei unterschiedlichen Lichtwellenlängen durchzuführen und diese Meßwerte insgesamt mittels eines geeigneten numerischen Algorithmus mit dem zugehörigen Konzentrationswert zu korrelieren. Geeignete Algorithmen sind bekannt und teilweise als Computerprogramme kommerziell erhältlich. Bei Verwendung eines derartigen Verfahrens kann es vorteilhaft sein, die Meßsignale für mindestens zwei unterschiedliche Wellenlängen und mindestens zwei unterschiedliche Meßtiefen unmittelbar (also ohne vorherige Quotientenbildung) als Eingangsvariable zu verwenden.

Obwohl die vorstehenden Erläuterungen sich überwiegend auf Glucose als Analyt beziehen, ist die Erfindung auch für andere Analyten anwendbar, für die folgenden Voraussetzungen gelten: Geringe spezifische optische Absorption des Analyten vor einem großen Absorptionshintergrund; Gesamtabsorption der Probe in dem untersuchten Wellenlängenbereich weitgehend konstant; starke wellenlängenabhängige Änderung der optischen Absorption (und folglich auch des Brechungsindex der interstitiellen Flüssigkeit) in dem untersuchten Wellenlängenbereich. Als möglicher Analyt mit derartigen Eigenschaften sei Alkohol erwähnt. Im Einzelfall kann durch eine experimentelle Erprobung der erfindungsgemäßen Anordnung über einen größeren Bereich von Lichtwellenlängen die Eignung der Erfindung für die jeweilige Analyse erprobt und ein optimaler Wellenlängenbereich festgestellt werden.

Figur 5 zeigt eine Ausführungsform, bei der als zusätzliches Tiefenselektionsmittel in dem Meßschritt eine Niederkohärenz-reflektometrische Messung durchgeführt wird. Ein solches Verfahren wird englisch auch als "LCI (low coherence interferometry) reflectometric measurement" oder auch als "optical coherence domain reflectometry (OCDR)" bezeichnet. Solche interferometrischen Meßverfahren sind für unterschiedliche Anwendungszwecke bekannt. Verwiesen sei insbesondere auf die Publikation: "Measurement of Optical Properties of Biological Tissues by Low-Coherence Reflectometry", von Schmitt et al., Applied Optics, 32 (1993), 6032-6042 sowie die WO 92/19930 und die bereits erwähnte WO 95/30368.

Für eine LCI-Messung ist erforderlich, daß ein Teil des von einem spektral breitbandig emittierenden Lichtsender abgestrahlten Lichts durch einen Strahlteiler abgeteilt, auf einem Referenzlichtweg einem optisch reflektierenden Element zugeführt, von diesem reflektiert und vor dem Detektor mit dem Meßlichtweg derartig zusammengefaßt wird, daß das Sekundärlicht und das Referenzlicht miteinander interferieren. Dabei mißt der Lichtempfänger ein Interferenzsignal, sofern die optische Lichtweglänge in dem Referenzarm (von dem Strahlteiler bis zu dem reflektierenden Element) sich maximal um die Kohärenzlänge der Lichtquelle von der optischen Weglänge des Meßlichtweges von dem Strahlteiler bis zu dem Reflexionspunkt in der Probe unterscheidet. Nur wenn diese Bedingung erfüllt ist, wird ein Interferenzsignal gemessen. Dies kann man nutzen, um die Untersuchung einer Probe auf eine bestimmte Meßtiefe d zu beschränken.

Bei der Ausführungsform nach Figur 5 ist ein zusätzlicher Lichtweg vorgesehen, der von in dem BSC 15 nach links reflektiertem Licht gebildet wird und den Referenzarm 30 einer Interferometeranordnung bildet. Das Referenzlicht wird an einem beweglichen Spiegel 31 reflektiert und fällt in den BSC 15 in der Gegenrichtung zurück, wobei es über den Sekundärlichtweg 19 zu der detektorseitigen Lichteintrittsöffnung (Lochblende 22) gelangt. Die Interferenzbedingung ist erfüllt, wenn der optische Lichtweg in dem Referenzarm 30 (bis zur Oberfläche des Spiegels 31) und in dem Probenarm 32 (bis zu der Meßtiefe d) übereinstimmen. Somit kann durch die Einstellung des Spiegels 31 die selektive Erfassung des aus der Meßtiefe d reflektierten Lichts verbessert werden. Nähere Einzelheiten über die meßtechnische Durchführung des LCI-Verfahrens sind den vorgenannten Literaturstellen zu entnehmen.

Eine weitere Besonderheit der in Figur 5 dargestellten Ausführungsform besteht darin, daß sowohl die Lichteinstrahlungsmittel 8 als auch die Detektionsmittel 9 jeweils Lichtleitfasern 33 bzw. 34 aufweisen, durch die das Licht von nicht dargestellten Lichtsendern zu dem BSC geleitet bzw. von dort zu ebenfalls nicht dargestellten Detektoren geleitet wird. Es ist für die Erfindung nicht von grundlegender Bedeutung, ob das Primärlicht (wie bei Figur 1) unmittelbar von den Lichtsendern 10 in die Meßanordnung eingestrahlt bzw. durch Detektoren 20 unmittelbar detektiert wird oder ob Lichtleiter (wie bei Figur 5) verwendet werden. Wesentlich ist nur, daß die effektive Lichtaustrittsöffnung 12, von der das Primärlicht in das optische System eintritt und die effektive Lichteintrittsöffnung 24 in dem Sekundärlichtweg 19 hinreichend kleine Abmessungen haben, um eine ausreichend scharfe Fokussierung sowohl in dem Primärlichtstrahlengang als auch in dem Sekundärlichtstrahlengang zu ermöglichen. Die Begrenzung der primärseitigen Lichtaustrittsöffnung und der sekundärseitigen Lichteintrittsöffnung kann dabei auf unterschiedliche Weise, beispielsweise wie dargestellt durch Lochblenden aber auch durch das entsprechend dimensionierte Austrittsende einer Lichtleitfaser oder durch die Größe der lichtempfindlichen Fläche eines Detektors realisiert sein.

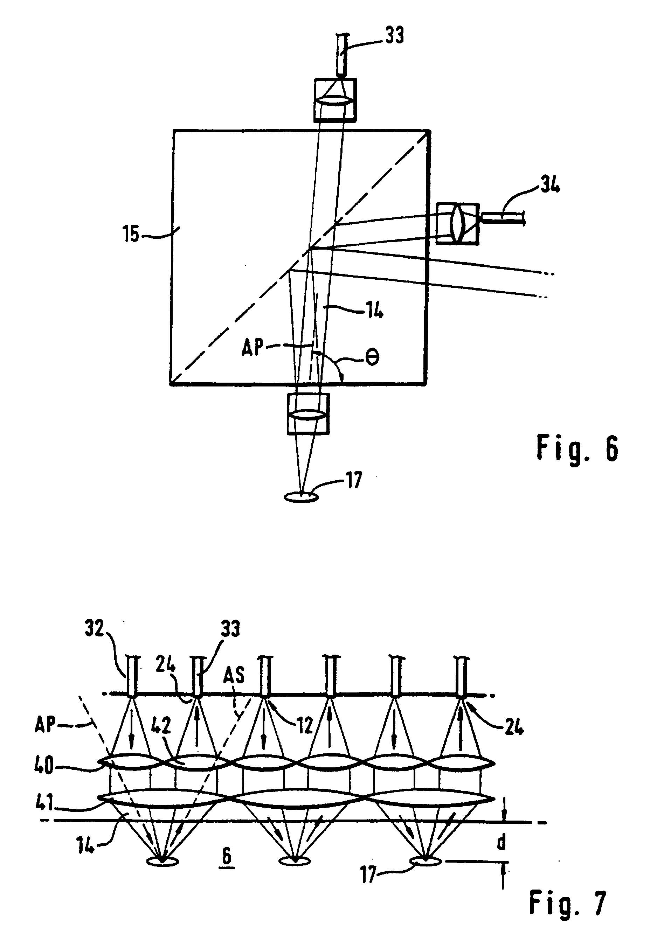

Ein Problem bei der in den Figuren 1 und 4 dargestellten Anordnung mit einem BSC sind Fresnel-Reflexionen, die durch den Brechungsindex-Sprung an den Grenzflächen des BSC verursacht werden. Dadurch wird Licht beispielsweise von der Grenzfläche 29 des BSC reflektiert, durch die das Meßlicht in die Probe austritt (Figur 5). Dieses starke Reflexionssignal kann leicht zu einer Übersteuerung des Detektors führen. Um dies zu vermeiden, wird bei der in Figur 6 dargestellten Ausführungsform eine Anordnung gewählt, bei der die Achse AP des Primärlicht-Strahles 14 unter einem Winkel θ, welcher kleiner ist als 90°, zu der entsprechenden Begrenzungsfläche 29 geneigt ist. Dadurch trifft der durch Fresnel-Reflexion spiegelnd reflektierte Lichtstrahl 35 nicht auf den Detektor 20.

Die Abweichung des Winkels θ ist in Figur 6 stark übertrieben dargestellt. In der Praxis genügt eine sehr kleine Abweichung um weniger als 1°, daß das spiegelnd reflektierte Licht nicht mehr auf die lichtdetektorseitige Lichteintrittsöffnung 24 fällt.

Während bei den Ausführungsformen der Figuren 1, 5 und 6 jeweils das aus dem Fokusbereich koaxial zurückreflektierte Sekundärlicht erfaßt wird (d.h. die Achse AP des Primärlichts und die optische Achse AS des Sekundärlichts zwischen dem optisch fokussierenden Element und dem Fokuspunkt übereinstimmt) zeigt Figur 7 eine Ausführungsform, bei der die optisch fokussierenden Elemente der Einstrahlungsmittel 8 und der Detektionsmittel 9 verschieden sind, wobei die optische Achse, auf der das Licht in die Probe eingestrahlt wird und die optische Achse, auf der das Licht detektiert wird, sich unterscheiden. Bei der dargestellten Ausführungsform durchdringt das von einer Lichtleitfaser 32 eingestrahlte Primärlicht für jeden Fokusbereich 17 eines Array jeweils eine erste Linse 40 und eine zweite Linse 41, die einen doppelt so großen Durchmesser wie die erste Linse hat. Das fokussierte Licht dringt asymmetrisch in die Matrix 6 ein. Nach Rückstreuung passiert das Sekundärlicht in der umgekehrten Reihenfolge die zweite Linse 41 und eine dritte Linse 42. Durch das System der Linsen 40,41,42 wird auch bei dieser Ausführungsform erreicht, daß das Primärlicht 14 auf den Fokusbereich 17 fokussiert und dieser Fokusbereich 17 auf die detektorseitige Lichteintrittsöffnung 24 abgebildet wird. Die Verwendung von insgesamt drei Linsen mit der dargestellten Anordnung hat den Vorteil, daß kommerziell erhältliche Microlinsen-Arrays verwendet werden können, während bei einer (grundsätzlich ebenfalls möglichen) schrägen (d.h. nicht zur Oberfläche 5 der Matrix 6 parallelen) Anordnung der Linsen eine Spezialanfertigung der Mikrolinsen-Anordnung erforderlich ist.

Bei der Ausführungsform gemäß Figur 7 können keine störenden Probleme mit Fresnel-Rückreflexion entstehen, da kein von einer geraden Fläche spiegelnd reflektiertes Licht den Detektor erreicht. Zwar führen optische Fehler der Linsen (insbesondere Abberationsfehler) zu Verzerrungen und damit zu einer weniger scharfen Definition des Fokusbereiches 17. Dies ist im Regelfall jedoch nicht störend. Erforderlichenfalls können bekannte optische Korrekturmaßnahmen angewandt werden.

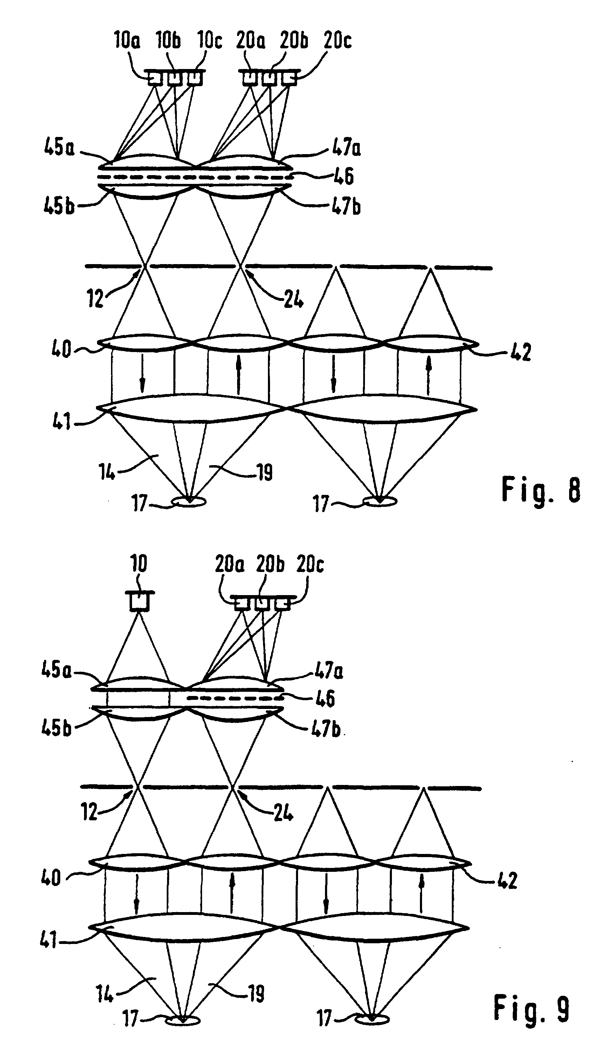

Die Figuren 8 und 9 zeigen zwei Möglichkeiten, bei einer Anordnung mit dem prinzipiellen Aufbau der Figur 7, eine Messung mit mehreren Lichtwellenlängen zu ermöglichen.

Bei Figur 8 werden als Primärlicht-Sender 10a, 10b, 10c drei Laserdioden verwendet, welche Licht mit unterschiedlichen Wellenlängen emittieren und zweckmäßigerweise auf einem gemeinsamen Substrat als integrierte Optik aufgebracht sind. Diese drei Lichtsender strahlen unter unterschiedlichen Einfallswinkeln auf eine Halblinse 45a ab, welche das Licht unter verschiedenen Winkeln kollimiert. Unterhalb der Halblinse 45a befindet sich ein optisches Gitter 46, dessen Gitterkonstante so angepaßt ist, daß die unter verschiedenen Winkeln auftreffenden kollimierten Strahlen unterschiedlicher Lichtwellenlänge in einen gemeinsamen (kollimierten) Strahl entlang der optischen Achse transformiert werden. Dieser wird durch eine zweite Halblinse 45b auf eine Lichtaustrittsöffnung 12 fokussiert.

Auf der Detektorseite befindet sich eine entsprechende Anordnung bestehend aus zwei Halblinsen 47a und 47b, dem Gitter 46 und Detektoren 20a,20b,20c für die unterschiedlichen Lichtwellenlängen. Die beiden Halblinsen 45a,45b, 47a,47b bilden mit dem Gitter 46 ein Sandwich, welches konstruktiv einfach mit Hilfe von Mikrolinsen-Strukturen aufgebaut werden kann.

Bei der Ausführungsform gemäß Figur 9 werden die drei Laserdioden durch eine einzige breitbandig abstrahlende Laserdiode 10 ersetzt. Die Wellenlängenselektivität wird hier durch das Gitter und die drei unter unterschiedlichen Winkeln hinter dem Gitter angeordneten Detektoren 20a,20b,20c, gewährleistet. Diese Anordnung ist bei geringerer Wellenlängen-Selektivität technisch einfacher als die von Figur 8.

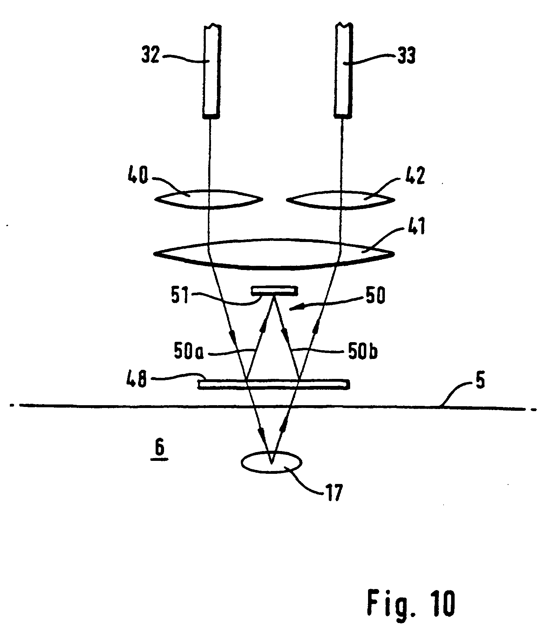

Figur 10 zeigt, in welcher Weise eine Niederkohärenz-reflektometrische Messung als zusätzliches Tiefenselektionsmittel bei einer Ausführungsform gemäß Figuren 7 bis 9 möglich ist. Dabei sind der Übersichtlichkeit halber nur die Zentralstrahlen der Strahlenbündel dargestellt. Als Strahlteiler zur Erzeugung eines Referenzstrahls dient hier ein horizontal verlaufenden halbdurchlässiger Spiegel 48. Der reflektierte Lichtstrahl 50a fällt auf einen Referenzreflektor 51, dessen Abstand von dem halbdurchlässigen Spiegel 48 dem Abstand des Fokusbereiches 17 von dem halbdurchlässigen Spiegel 48 entspricht. Aus Symmetriegründen wird das auf den Referenzspiegel 51 auftreffende Licht ebenso wie das in die Probe 6 eingestrahlte Licht fokussiert. Das von dem Referenzreflektor 51 reflektierte Licht fällt als Lichtstrahl 50b auf den halbdurchlässigen Spiegel 48 zurück und wird von dort in Richtung auf den Reflektor reflektiert. Die Strahlen 50a und 50b bilden bei dieser Anordnung den Referenzarm 50 der Interferometeranordnung. Eine Modulation des Referenzlichtes kann durch einen vibrierenden Referenzreflektor 51 bewirkt werden. Alternativ kann ein LCD-Element im Lichtstrahl derartig angeordnet sein und mit einer geeigneten Modulationsfrequenz angesteuert werden, daß das Referenzlicht moduliert wird.

## Patentansprüche

1. Verfahren zur Bestimmung eines Analyten in einer streuenden, insbesondere biologischen, Matrix, bei welchem
in einem Detektionsschritt Detektionsmessungen durchgeführt werden, bei denen Licht durch eine die streuende Matrix (6) begrenzende Grenzfläche als Primärlicht in die Matrix (6) eingestrahlt und aus der streuenden Matrix (6) durch die Grenzfläche austretendes Licht als Sekundärlicht mittels eines Detektors (20) detektiert wird, um eine durch die Wechselwirkung des Lichts mit der Matrix (6) veränderliche, meßbare physikalische Eigenschaften des Lichtes als Meßgröße zu bestimmen, und
in einem Auswerteschritt eine Information über die Gegenwart des Analyten in der Matrix (6) auf Basis eines in dem Detektionsschritt gemessenen Meßwertes der Meßgröße ermittelt wird,
wobei mittels einer konfokalen Meßanordnung
das Primärlicht (14) mittels eines optisch fokussierenden Elementes (16;40,41) auf einen in der Matrix (6) in einer Fokustiefe (d) unter der Grenzfläche liegenden Fokusbereich (17) fokussiert wird, und
der Fokusbereich (17) mittels eines optisch fokussierenden Elementes (16,21;41,42) auf eine im Lichtweg des Sekundärlichtes (19) zu dem Detektor angeordnete Lichteintrittsöffnung (24) abgebildet und dadurch die Detektion des Sekundärlichtes (19) auf den Fokusbereich (17) konzentriert wird,
**dadurch gekennzeichnet, daß**
zur Gewinnung eines Meßsignals, das eine selektive Analyse eines Analyten ermöglicht, der einen geringen Anteil an der optischen Gesamtabsorption der Matrix hat,
mittels eines zusätzlichen Tiefenselektionsmittels selektiv Sekundärlicht, das in einer definierten Meßtiefe unter der Grenzfläche (5) reflektiert wurde, von dem Detektor (20) detektiert wird, wobei die definierte Meßtiefe mit der Fokustiefe (d) übereinstimmt,
so daß eine selektive Erfassung des in einer mit der Fokustiefe übereinstimmenden Meßtiefe in der streuenden Matrix reflektierten Lichtes durch zwei in Kombination miteinander angewandte Selektionsverfahren erfolgt.

2. Verfahren nach Anspruch 1, **dadurch gekennzeichnet, daß** mindestens zwei Detektionsmessungen mit unterschiedlichen Lichtwellenlängen durchgeführt werden.

3. Verfahren nach Anspruch 2, **dadurch gekennzeichnet, daß** in dem Auswerteschritt ein Quotient der Meßwerte bei den zwei unterschiedlichen Wellenlängen bestimmt und die Information über die Gegenwart des Analyten auf Basis des Quotienten ermittelt wird.

4. Verfahren nach einem der vorgehenden Ansprüche, **dadurch gekennzeichnet, daß** das Primärlicht mittels eines Array optisch fokussierender Elemente (16; 40,41) auf eine Mehrzahl von in der gleichen Fokustiefe (d) unter der Grenzfläche liegenden Fokusbereichen (17) fokussiert wird und die Mehrzahl der Fokusbereiche (17) mittels einer entsprechenden Anzahl optisch fokussierender Elemente (16,21; 41,42) auf jeweils eine im Lichtweg zu dem Detektor angeordnete Lichteintrittsöffnung (24) abgebildet wird.

5. Verfahren nach Anspruch 4, **dadurch gekennzeichnet, daß** das Primärlicht (14) auf mindestens zehn verschiedene Fokusbereiche (17) fokussiert wird.

6. Verfahren nach einem der vorhergehenden Ansprüche, **dadurch gekennzeichnet, daß** die mit der Fokustiefe übereinstimmende Meßtiefe (d) mindestens 0,3 mm und höchstens 1,5 mm beträgt.

7. Verfahren nach einem der vorhergehenden Ansprüche, **dadurch gekennzeichnet, daß** mindestens zwei Detektionsmessungen durchgeführt werden, bei denen die mit der Fokustiefe (d) übereinstimmende Meßtiefe unterschiedlich ist.

8. Verfahren nach Anspruch 7, **dadurch gekennzeichnet, daß** sich die mit der Fokustiefe übereinstimmende Meßtiefe bei den mindestens zwei Detektionsmessungen um mindestens 0,3 mm unterscheidet.

9. Verfahren nach einem der vorhergehenden Ansprüche, **dadurch gekennzeichnet, daß** als Tiefenselektionsmittel in dem Meßschritt eine Niederkohärenz-reflektometrische Messung durchgeführt wird.

10. Verfahren nach einem der vorhergehenden Ansprüche, **dadurch gekennzeichnet, daß** als Tiefenselektionsmittel in dem Meßschritt das Primärlicht in Form eines kurzen Lichtimpulses eingestrahlt und das Sekundärlicht innerhalb eines definierten Zeitfensters gemessen wird.

11. Verfahren nach einem der vorhergehenden Ansprüche, **dadurch gekennzeichnet, daß** der Analyt Glucose ist.

12. Verfahren nach Anspruch 11, **dadurch gekennzeichnet, daß** die Wellenlänge des Lichtes bei mindestens einer der Detektionsmessungen zwischen 2,0 µm und 2,4 µm liegt.

13. Vorrichtung zur Durchführung des Verfahrens nach einem der vorhergehenden Ansprüche mit
einem Meßkopf (2) mit einer Probenkontaktfläche (4) zum Anlegen an eine Grenzfläche (5) der streuenden Matrix (6),
Lichteinstrahlungsmitteln (8) mit einem Lichtsender (10) zum Einstrahlen von Primärlicht (14) in die streuende Matrix (6) durch die Probenkontaktfläche (4) und die Grenzfläche (5),
Detektionsmitteln mit einem Detektor (20) zum Detektieren von durch die Grenzfläche (5) und die Probenkontaktfläche (4) aus der streuenden Matrix (6) austretendem Sekundärlicht (19) und
Auswertemitteln (26,27) zur Ermittlung der Information über die Gegenwart des Analyten in der Matrix (6) aus dem Meßsignal des Detektors (20),
wobei die Einstrahlungsmittel (8) und die Detektionsmittel (9) jeweils ein optisch fokussierendes Element aufweisen, das optisch fokussierende Element (13,16;40,41) der Einstrahlungsmittel (8) das Primärlicht (14) auf einen in der Matrix (6) in einer Fokustiefe (d) unter der Grenzfläche (5) liegenden Fokusbereich (17) fokussiert und das optisch fokussierende Element der Detektionsmittel (9) den Fokusbereich auf eine in dem Lichtweg des Sekundärlichtes (19) zu dem Detektor (20) angeordnete Lichteintrittsöffnung (24) abbildet, wodurch die Detektion des Sekundärlichtes (19) auf den Fokusbereich (17) konzentriert wird,
**dadurch gekennzeichnet, daß**
zur Gewinnung eines Meßsignals, das eine selektive Analyse eines Analyten ermöglicht, der einen geringen Anteil an der optischen Gesamtabsorption der Matrix hat,
ein zusätzliches Tiefenselektionsmittel vorgesehen ist, um selektiv Sekundärlicht, das in einer definierten Meßtiefe unter der Grenzfläche (5) reflektiert wurde, mittels des Detektors (20) zu detektieren, wobei die definierte Meßtiefe mit der Fokustiefe (d) übereinstimmt,
so daß eine selektive Erfassung des in einer mit der Fokustiefe übereinstimmenden Meßtiefe in der streuenden Matrix reflektierten Lichtes durch zwei in Kombination miteinander angewandte Tiefenselektionsmittel erfolgt.

14. Vorrichtung nach Anspruch 13, **dadurch gekennzeichnet, daß** der Durchmesser der Lichteintrittsöffnung weniger als 0,1 mm beträgt.

15. Vorrichtung nach Anspruch 13, **dadurch gekennzeichnet, daß** sowohl die Einstrahlungsmittel (8) als auch die Detektionsmittel (9) ein Array optisch fokussierender Elemente aufweisen, durch die das Primärlicht (14) auf eine Mehrzahl von Fokusbereichen (17) fokussiert wird und die Mehrzahl der Fokusbereiche (17) auf jeweils eine Lichteintrittsöffnung (24) vor einem Detektor (20) abgebildet wird.

16. Vorrichtung nach Anspruch 15, **dadurch gekennzeichnet, daß** das Array optisch fokussierender Elemente ein Mikrolinsen-Array (16a,21a) ist.

17. Vorrichtung nach Anspruch 15 oder 16, **dadurch gekennzeichnet, daß** eine Mehrzahl von Detektoren (20) auf einem Halbleiterchip (23) monolitisch integriert ist.

18. Vorrichtung nach einem der Ansprüche 15 bis 17, **dadurch gekennzeichnet, daß** eine Mehrzahl von Lichtsendern (10) auf einem Halbleiterchip (11) monolitisch integriert ist.

19. Vorrichtung nach einem der Ansprüche 13 bis 18, **dadurch gekennzeichnet, daß** das gleiche optisch fokussierende Element (16) sowohl Teil der Einstrahlungsmittel (8) als auch Teil der Detektionsmittel (9) ist, so daß die optische Achse (AP) des Primärlichtes (17) und die optische Achse (AS) des Sekundärlichtes (19) zwischen dem optisch fokussierenden Element (16) und dem Fokusbereich (17) übereinstimmt und auf der von der Matrix (6) abgewandten Seite des fokussierenden Elementes (16) ein Strahlteiler angeordnet ist, durch den das Sekundärlicht (19) zu dem Detektor (20) abgezweigt wird.

20. Vorrichtung nach einem der Ansprüche 13 bis 18, **dadurch gekennzeichnet, daß** die Einstrahlungsmittel (8) und die Detektionsmittel (9) getrennte optisch fokussierende Elemente (40 bzw. 42) aufweisen, wobei die optische Achse (AP), auf der das Primärlicht (14) in die Matrix (6) eingestrahlt wird, und die optische Achse (AS), auf der das Sekundärlicht (19) detektiert wird, verschieden sind.

21. Vorrichtung nach einem der Ansprüche 13 bis 20, **dadurch gekennzeichnet, daß** sie als Tiefenselektionsmittel eine Niederkohärenz-reflektometrische Meßeinrichtung einschließt.

22. Vorrichtung nach einem der Ansprüche 13 bis 20, **dadurch gekennzeichnet, daß** sie als Tiefenselektionsmittel eine Einrichtung einschließt, um das Primärlicht in Form eines kurzen Lichtimpulses einzustrahlen und das Sekundärlicht innerhalb eines definierten Zeitfensters zu messen.

## Claims

1. Method for determining an analyte in a scattering, in particular biological, matrix, in which
in a detection step detection measurements are carried out in which light is irradiated through an interface limiting the scattering (6) as primary light into the matrix (6) and light leaving matrix the scattering matrix (6) through the interface is detected as secondary light by means of a detector (20), in order to determine as a measurement quantity a measurable physical property of the light which is variable due to the interaction of the light with the matrix (6), and
in an evaluation step information on the presence of the analyte in the matrix (6) is determined on the basis of a measured value of the measurement quantity measured in the detection step,
wherein by means of a confocal measuring arrangement
the primary light (14) is focussed by means of an optically focussing element (16; 40, 41) onto a region of focus (17) lying in the matrix (6) at a depth of focus (d) below the interface, and
the region of focus (17) is imaged by means of an optically focussing element (16, 21; 41, 42) onto a light entry aperture (24) arranged in the light path of the secondary light (19) to the detector, the detection of the secondary light (19) thereby being concentrated on the region of focus (17),
**characterized in that**
for generating a measurement signal allowing a selective analysis of an analyte which causes only a small share of the total optical absorption,
light reflected at a defined measuring depth below the interface (5) is detected selectively by the detector (20) as secondary light by means of an additional depth selection means, the defined measuring depth coinciding with the depth of focus (d),
so that the light reflected in the scattering matrix at a measuring depth coinciding with the depth of focus is selectively detected by means of two selection methods combined with each other.

2. Method according to claim 1, **characterized in that** at least two detection measurements are made with different light wavelengths.

3. Method according to claim 2, **characterized in that** in the evaluation step a quotient of the measured values at the two different wavelengths is obtained and the information on the presence of the analyte is determined on the basis of the quotient.

4. Method according to any one of the preceding claims, **characterized in that** the primary light is focussed by means of an array of optically focussing elements (16; 40, 41) onto a plurality of regions of focus (17) located at the same depth of focus (d) below the interface and the plurality of the regions of focus (17) is imaged by means of a corresponding number of optically focussing elements (16, 21; 41, 42) onto respective light entry apertures (24) arranged in the light path to the detector.

5. Method according to claim 4, **characterized in that** the primary light (14) is focussed onto at least ten different regions of focus (17).

6. Method according to any one of the preceding claims, **characterized in that** the measuring depth (d) coinciding with the depth of focus is at least 0.3 mm and at most 1.5 mm.

7. Method according to any one of the preceding claims, **characterized in that** at least two detection measurements are made, in which the measuring depth coinciding with the depth of focus (d) is different.

8. Method according to claim 7, **characterized in that** the measuring depth coinciding with the depth of focus differs by at least 0.3 mm in the at least two detection measurements.

9. Method according to any one of the preceding claims, **characterized in that** in the measuring step a low coherence reflectometric measurement is performed as a depth selection means.

10. Method according to any one of the preceding claims, **characterized in that** as a depth selection means in the measuring step the primary light is irradiated in the form of a short light pulse and the secondary light is measured within a defined time window.

11. Method according to any one of the preceding claims, **characterized in that** the analyte is glucose.

12. Method according to claim 11, **characterized in that** the wavelength of the light in at least one of the detection measurements is between 2.0 µm and 2.4 µm.

13. Apparatus for carrying out the method according to any one of the preceding claims comprising
a measuring head (2) with a sample contact surface (4) adapted to be placed against an interface (5) of the scattering matrix (6),
light irradiation means (8) with a light transmitter (10) for irradiating primary light (14) into the scattering matrix (6) through the sample contact surface (4) and the interface (5),
detection means with a detector (20) for detecting secondary light (19) leaving the scattering matrix (6) through the interface (5) and the sample contact surface (4) and
evaluation means (26, 27) for deriving the information on the presence of the analyte in the matrix (6) from the measuring signal of the detector (20),
in which the irradiation means (8) and the detection means (9) each comprise an optically focussing element, the optically focussing element (13, 16; 40, 41) of the irradiation means (8) focusses the primary light (14) onto a region of focus (17) lying in the matrix (6) at a depth of focus (d) below the interface (5), and the optically focussing element of the detection means (9) images the region of focus onto a light entry aperture (24) arranged in the light path of the secondary light (19) to the detector (20), whereby the detection of the secondary light (19) is concentrated on the region of focus (17),
**characterized in that**
for generating a measurement signal allowing a selective analysis of an analyte which causes only a small share of the total optical absorption,
an additional depth selection means is provided for selectively detecting light, by means of a detector 20, as secondary light reflected at a defined measuring depth, the measuring depth coinciding with the depth of focus (d),
so that the light reflected in the scattering matrix at a measuring depth coinciding with the depth of focus is selectively detected by means of two depth selection methods combined with each other.

14. Apparatus according to claim 13, **characterized in that** the diameter of the light entry aperture is less than 0.1 mm.

15. Apparatus according to claim 13, **characterized in that** both the irradiation means (8) and the detection means (9) comprise an array of optically focussing elements by means of which the primary light (14) is focussed onto a plurality of regions of focus (17) and the plurality of the regions of focus (17) is imaged onto respective light entry apertures (24) in front of a detector (20).

16. Apparatus according to claim 15, **characterized in that** the array of optically focussing elements is a microlens array (16a, 21a).

17. Apparatus according to claim 15 or 16, **characterized in that** a plurality of detectors (20) is integrated monolithically on a semiconductor chip (23).

18. Apparatus according to any one of claims 15 to 17, **characterized in that** a plurality of light transmitters (10) is integrated monolithically on a semiconductor chip (11).

19. Apparatus according to any one of claims 13 to 18, **characterized in that** the same optically focussing element (16) forms part both of the irradiation means (8) and of the detection means (9), so that the optical axis (AP) of the primary light (17) and the optical axis (AS) of the secondary light (19) coincide between the optically focussing element (16) and the region of focus (17), and
on the side of the focussing element (16) facing away from the matrix (6) a beam splitter is disposed by means of which the secondary light (19) is branched to the detector (20).

20. Apparatus according to any one of claims 13 to 18, **characterized in that** the irradiation means (8) and the detection means (9) comprise separate optically focussing elements (40 and 42), wherein the optical axis (AP), on which the primary light (14) is irradiated into the matrix (6), and the optical axis (AS), on which the secondary light (19) is detected, are different.

21. Apparatus according to any one of claims 13 to 20, **characterized in that** it includes as a depth selection means a low coherence reflectometric measuring device.

22. Apparatus according to any one of claims 13 to 20, **characterized in that** it includes as a depth selection means a device for irradiating the primary light in the form of a short light pulse and for measuring the secondary light within a defined time window.

## Revendications

1. Procédé de détermination d'un analyte contenu dans une matrice de diffusion, en particulier biologique, dans lequel
à une étape de détection des mesures de détection sont effectuées, dans lesquelles de la lumière est incidente à travers une surface limite délimitant la matrice de diffusion (6) en tant que lumière primaire dans la matrice (6) et la lumière se dégageant de la matrice de diffusion (6) à travers la surface limite est détectée comme lumière secondaire à l'aide d'un détecteur (20), afin de déterminer en tant que grandeur mesurée une propriété physique mesurable, variable par l'interaction de la lumière avec la matrice (6), et
à une étape d'évaluation une information concernant la présence de l'analyte dans la matrice (6) est établie sur la base d'une valeur indiquée, mesurée à l'étape de détection, de la grandeur mesurée,
dans lequel à l'aide d'un dispositif de mesure à foyer commun
la lumière primaire (14) est focalisée à l'aide d'un élément de focalisation optique (16 ; 40, 41) sur une zone focale (17) se trouvant dans la matrice (6) à une profondeur focale (d) sous la surface limite, et
la zone focale (17) est reproduite à l'aide d'un élément de focalisation optique (16, 21 ; 41, 42) sur une ouverture d'entrée de la lumière (24) disposée dans le trajet lumineux de la lumière secondaire (19) en direction dû détecteur et de ce fait la détection de la lumière secondaire (19) est concentrée sur la zone focale (17),
**caractérisé en ce que**
pour obtenir un signal de mesure permettant une analyse sélective d'un analyte, qui participe peu à l'absorption globale optique de la matrice,
à l'aide d'un moyen de sélection supplémentaire des profondeurs, une lumière secondaire, qui a été réfléchie à une profondeur mesurée définie sous la surface limite (5), est détectée sélectivement par le détecteur (20), la profondeur mesurée définie coïncidant avec la profondeur focale (d), si bien qu'une saisie sélective de la lumière refléchie dans la matrice de diffusion à une profondeur mesurée coïncidant avec la profondeur focale a lieu grâce à deux procédés de sélection appliqués ensemble de façon combinée.

2. Procédé selon la revendication 1, **caractérisé en ce qu'**au moins deux mesures de détection sont effectuées avec différentes longueurs d'ondes lumineuses.

3. Procédé selon la revendication 2, **caractérisé en ce qu'**à l'étape d'évaluation un quotient des valeurs mesurées avec les deux longueurs d'onde différentes est déterminé et l'information concernant la présence de l'analyte est établie sur la base du quotient.

4. Procédé selon l'une des revendications précédentes, **caractérisé en ce que** la lumière primaire est focalisée à l'aide d'un réseau d'éléments de focalisation optique (16 ; 40, 41) sur une majeure partie des zones focales (17) situées à la même profondeur focale (d) sous la surface limite et la majeure partie des zones focales (17) est reproduite à l'aide d'un nombre correspondant d'éléments de focalisation optique (16, 21 ; 41, 42) sur à chaque fois une ouverture d'entrée de lumière (24) disposée dans le trajet lumineux en direction du détecteur.

5. Procédé selon la revendication 4, **caractérisé en ce que** la lumière primaire (14) est focalisée sur au moins dix zones focales différentes (17).

6. Procédé selon l'une des revendications précédentes, **caractérisé en ce que** la profondeur mesurée (d) coïncidant avec la profondeur focale est d'au moins 0,3 mm et de 1,5 mm au maximum.

7. Procédé selon l'une des revendications précédentes, **caractérisé en ce qu'**au moins deux mesures de détection sont effectuées, dans lesquelles la profondeur mesurée coïncidant avec la profondeur focale (d) est différente.

8. Procédé selon la revendication 7, **caractérisé en ce que** la profondeur mesurée coïncidant avec la profondeur focale se différencie d'au moins 0,3 mm dans au moins deux mesures de détection.

9. Procédé selon l'une des revendications précédentes, **caractérisé en ce que** comme moyen de sélection des profondeurs, à l'étape de mesure, une mesure réflectométrique de cohérence basse est effectuée.

10. Procédé selon l'une des revendications précédentes, **caractérisé en ce que** comme moyen de sélection des profondeurs à l'étape de mesure, la lumière primaire est incidente sous forme d'une impulsion lumineuse courte et la lumière secondaire est mesurée au sein d'un intervalle de temps défini.

11. Procédé selon l'une des revendications précédentes, **caractérisé en ce que** l'analyte est du glucose.

12. Procédé selon la revendication 11, **caractérisé en ce que** la longueur d'onde de la lumière se situe dans au moins l'une des mesures de détection entre 2,0 µm et 2,4 µm.

13. Dispositif d'exécution du procédé selon l'une des revendications précédentes comprenant
une tête de mesure (2) avec une surface de contact d'essai (4) pour application sur une surface limite (5) de la matrice de diffusion (6),
des moyens de radiation incidente de lumière (8) avec un émetteur de lumière (10) pour rendre incidente la lumière primaire (14) dans la matrice de diffusion (6) à travers la surface de contact d'essai (4) et la surface limite (5),
des moyens de détection avec un détecteur (20) pour détecter la lumière secondaire (19) se dégageant de la matrice de diffusion (6) à travers la surface limite (5) et la surface de contact d'essai (4) et
des moyens d'évaluation (26, 27) pour établir l'information concernant la présence de l'analyte dans la matrice (6) à partir du signal de mesure du détecteur (20),
les moyens de radiation incidente (8) et les moyens de détection (9) présentant à chaque fois un élément de focalisation optique, l'élément de focalisation optique (13, 16 ; 40, 41) des moyens de radiation incidente (8) focalise la lumière primaire (14) sur une zone focale (17) située dans la matrice (6) à une profondeur focale (d) sous la surface limite (5) et l'élément de focalisation optique des moyens de détection (9) reproduit la zone focale sur une ouverture d'entrée de lumière (24) disposée dans le trajet lumineux de la lumière secondaire (19) en direction du détecteur (20), la détection de la lumière secondaire (19) étant concentrée sur la zone focale (17),
**caractérisé en ce que** pour obtenir un signal de mesure permettant une analyse sélective d'un analyte, qui participe peu à l'absorption globale optique de la matrice,
un moyen de sélection supplémentaire des profondeurs est prévu, pour détecter sélectivement une lumière secondaire, qui a été réfléchie à une profondeur mesurée définie sous la surface limite (5), au moyen du détecteur (20), la profondeur mesurée définie coïncidant avec la profondeur focale (d),
si bien qu'une saisie sélective de la lumière réfléchie à une profondeur mesurée coïncidant avec la profondeur focale dans la matrice de diffusion a lieu grâce à deux moyens de sélection des profondeurs appliqués ensemble de façon combinée.

14. Dispositif selon la revendication 13, **caractérisé en ce que** le diamètre de l'ouverture d'entrée de la lumière est inférieur à 0,1 mm.

15. Dispositif selon la revendication 13, **caractérisé en ce que** non seulement les moyens de radiation incidente (8) mais aussi les moyens de détection (9) présentent un réseau d'éléments de focalisation optique, par lesquels la lumière primaire (14) est focalisée sur une pluralité de zones focales (17) et la pluralité de zones focales (17) est reproduite sur à chaque fois une ouverture d'entrée de lumière (24) devant un détecteur (20).

16. Dispositif selon la revendication 15, **caractérisé en ce que** le réseau d'éléments de focalisation optique est un réseau à micro-lentilles (16a, 21a).

17. Dispositif selon la revendication 15 ou 16, **caractérisé en ce qu'**une pluralité de détecteurs (20) est intégrée de façon monolithique sur une puce semi-conductrice (23).

18. Dispositif selon l'une des revendications 15 à 17, **caractérisé en ce qu'**une pluralité d'émetteurs de lumière (10) est intégrée de façon monolithique sur une puce semi-conductrice (11).

19. Dispositif selon l'une des revendications 13 à 18, **caractérisé en ce que** le même élément de focalisation optique (16) fait non seulement partie des moyens de radiation incidente (8) mais également partie des moyens de détection (9), de sorte que l'axe optique (AP) de la lumière primaire (17) et l'axe optique (AS) de la lumière secondaire (19) coïncident entre l'élément de focalisation optique (16) et la zone focale (17) et
sur le côté opposé à la matrice (6) de l'élément de focalisation (16) est disposé un diviseur de rayon, par lequel la lumière secondaire (19) est divisée en direction du détecteur (20).

20. Dispositif selon l'une des revendications 13 à 18, **caractérisé en ce que** les moyens de radiation incidente (8) et les moyens de détection (9) présentent des moyens de focalisation optique distincts (40 resp. 42), l'axe optique (AP), sur lequel la lumière primaire (14) est rendue incidente dans la matrice (6), et l'axe optique (AS), sur lequel la lumière secondaire (19) est détecté, étant différents.

21. Dispositif selon l'une des revendications 13 à 20, **caractérisé en ce qu'**il renferme comme moyen de sélection des profondeurs un dispositif de mesure réflectométrique de cohérence basse.

22. Dispositif selon l'une des revendications 13 à 20, **caractérisé en ce qu'**il renferme comme moyen de sélection des profondeurs un dispositif visant à rendre incidente la lumière primaire sous forme d'une impulsion lumineuse courte et à mesurer la lumière secondaire au sein d'un intervalle de temps défini.
